(19) Europäisches Patentamt
European Patent Office
Office européen des brevets

(11) Numéro de publication: **0 511 888 B1**

(12) **FASCICULE DE BREVET EUROPEEN**

(45) Date de publication de fascicule du brevet: **05.07.95**

(51) Int. Cl.[6]: **C07C 323/12**, C07C 319/20, C10M 135/24, //C10N30/06

(21) Numéro de dépôt: **92401022.6**

(22) Date de dépôt: **10.04.92**

# (54) Nouveaux composés carboxyliques (poly)sulfures, leur préparation et leur utilisation notamment comme additifs pour huiles lubrifiantes.

(30) Priorité: **23.04.91 FR 9105091**

(43) Date de publication de la demande:
**04.11.92 Bulletin 92/45**

(45) Mention de la délivrance du brevet:
**05.07.95 Bulletin 95/27**

(84) Etats contractants désignés:
**BE DE DK ES GB IT NL SE**

(56) Documents cités:
**BE-A- 606 732**
**FR-A- 1 262 508**
**GB-A- 960 184**
**US-A- 2 743 293**
**US-A- 4 615 836**

(73) Titulaire: **INSTITUT FRANCAIS DU PETROLE**
**4, Avenue de Bois Préau**
**F-92502 Rueil-Malmaison (FR)**

(72) Inventeur: **Aberkane, Ourida**
**8, Boucle de la Seille**
**F-57290 Fameck (FR)**
Inventeur: **Born, Maurice**
**72, rue du Vieux Pont**
**F-92000 Nanterre (FR)**
Inventeur: **Mieloszynski, Jean-Luc**
**286, rue Pont à Mousson**
**F-57158 Montigny les Metz (FR)**
Inventeur: **Paquer, Daniel**
**Résidence Minerve,**
**2, rue d'Anvers**
**F-54500 Vandoeuvre (FR)**
Inventeur: **Parc, Guy**
**27, rue du Chateau**
**F-92500 Rueil Malmaison (FR)**

EP 0 511 888 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouveaux composés carboxyliques, acides et esters, (poly)sulfurés, leur préparation et leur utilisation comme additifs pétroliers, notamment comme additifs antiusure et extrême-pression pour huiles lubrifiantes.

L'utilisation d'additifs antiusure et extrême-pression notamment dans les huiles moteurs, les fluides de transmission, les fluides hydrauliques se pratique depuis plusieurs décennies. De nombreux types d'additifs ont ainsi été développés et plusieurs d'entre eux ont permis de réduire très sensiblement la détérioration des mécanismes et ont, de ce fait, permis de prolonger leur durée de vie.

Parmi les nombreux additifs antiusure et extrême-pression qui ont été étudiés, les dialkyl- et diaryldithiophosphates et les dialkyldithiocarbamates métalliques (ceux de zinc notamment), les alkylthiophosphates, le tricrésylphosphate, le didodécylphosphate, les terpènes sulfurés, l'huile de spermacéti soufrée et divers composés chlorés se sont montrés les plus actifs et ont connu un développement industriel important. Certains d'entre eux sont décrits dans les brevets US-A-2.364.283, 2.364.284, 2.365.938, 2.410.650, 2.438.876 et 3.190.833. Il s'agit généralement de composés renfermant des hétéroatomes tels que le soufre et le phosphore, soit seuls (par exemple le tricrésylphosphate, les terpènes soufrés, les dithiocarbamates), soit en association (par exemple des dialkyldithiophosphates métalliques, des alkylthiophosphates).

Parmi les composés soufrés, certains peuvent renfermer des fonctions chimiques conférant au composé dans son utilisation comme additif des propriétés physico-chimiques ou des performances mécaniques améliorées. Il s'agit entre autres des fonctions phénol, nitrile, sulfoxyde, xanthate, etc. A cet égard, on peut citer les brevets américains US-A-3.434.852, 3.984.336 et 3.994.923.

La quantité de soufre contenue dans la molécule de ces composés soufrés est généralement fixée par la stoechiométrie des réactions mises en oeuvre lors de leur synthèse, ce qui leur confère des propriétés antiusure et extrême-pression sur lesquelles on ne peut exercer aucune modification.

Or, on a maintenant découvert de nouveaux composés soufrés, utilisables comme additifs pour lubrifiants, pour lesquels il est possible de modifier la proportion de soufre qu'ils contiennent dans leur molécule et donc de moduler à volonté leurs propriétés antiusure et extrême-pression.

Les composés de la présente invention sont définis comme étant (poly)sulfurés, ce qui signifie que, dans leur molécule, ils peuvent contenir soit un ou plusieurs atomes de soufre isolés (composés monosulfurés!, soit un ou plusieurs enchaînements de plusieurs atomes de soufre (composés polysulfurés).

Les composés carboxyliques (poly)sulfurés de l'invention peuvent être définis d'une manière générale par le fait qu'ils répondent à la formule suivante :

$$[R^3\text{-}OC(O)\text{-}]_q R^2\{\text{-}\ C(O)O\text{-}(CH_2)_m\text{-}CH_{3-z}\ [\text{-}(CH_2)_n\text{-}S_x\text{-}R^1]_z\}_p \quad (1)$$

dans laquelle $R^1$ représente un radical aliphatique, monovalent, saturé ou insaturé, de 1 à 30, et de préférence de 1 à 12 atomes de carbone ; x est un nombre égal ou supérieur à 1 ; z est égal à 1 ou 2 ; lorsque z est égal à 1, n est un nombre entier de 0 à 5 et m est un nombre entier de 0 à 5, avec $m+n$ entier de 1 à 10 ; lorsque z est égal à 2, n est un nombre entier de 1 à 5 et m un nombre entier de 0 à 5, avec $m+n$ entier de 1 à 10 ; $R^2$ est un radical hydrocarboné de 1 à 51 atomes de carbone et de valence $p+q$ ; $R^3$ est un atome d'hydrogène ou un radical aliphatique monovalent de 1 à 30 atomes de carbone ; p est un nombre entier de 1 à 4 ; et q est un nombre entier de 0 à 3, avec $p+q$ entier de 1 à 4.

Ces composés consistent donc en des esters formés entre un acide mono ou polycarboxylique de formule : $R^2\ [C(O)OH]_{p+q}$ (2) et un monoalcool (poly)sulfuré de formule générale: $[R^1\text{-}S_x\text{-}(CH_2)_n\text{-}]_z\ CH_{3-z}\text{-}(CH_2)_m\text{-}OH$ (3) et éventuellement un monalcool aliphatique $R^3OH$ (4) où $R^1$, $R^2$, $R^3$, x, z, n, m, p et q ont les mêmes significations que précédemment ; au moins une des fonctions carboxyliques de l'acide de formule (2) étant estérifiée par un alcool (poly)sulfuré de formule (3).

On considère plus particulièrement dans l'invention les composés carboxyliques consistant en des esters d'acides monocarboxyliques, ou en des esters d'acides polycarboxyliques (di-, tri, ou tétracarboxyliques) dont toutes les fonctions carboxyliques sont estérifiées par un monoalcool (poly)sulfuré de formule (3). Ces esters correspondent donc à la formule (1) dans laquelle q est égal à zéro.

Les acides carboxyliques de formule (2) peuvent être choisis parmi les acides mono ou polycarboxyliques aliphatiques de 2 à 54 atomes de carbone, cycloaliphatiques ou aromatiques de 6 à 20 atomes de carbone. Il peut s'agir plus particulièrement d'acides mono-, di-, tri- ou tétracarboxyliques.

Comme exemples de tels acides on peut citer:

- les acides monocarboxyliques aliphatiques saturés, tels que l'acide acétique, l'acide propionique, l'acide butyrique, l'acide caproïque, l'acide caprylique, l'acide caprique, l'acide palmitique ou l'acide

stéarique ;

- les acides monocarboxyliques aliphatiques insaturés, tels que l'acide acrylique, l'acide méthacrylique, les acides oléique, ricinoléique, linoléique, linolénique ou l'acide érucique ;
- les acides dicarboxyliques aliphatiques, saturés ou insaturés, tels que l'acide oxalique, l'acide malonique, l'acide succinique, l'acide maléique, l'acide adipique, l'acide azélaïque ou les acides dimères d'acides oléique, linoléique et/ou linolénique ;
- les acides tricarboxyliques aliphatiques, saturés ou insaturés, tels que par exemple les acides trimères d'acides oléique, linoléique et/ou linolénique ;
- les acides monocarboxyliques cycloaliphatiques , tels que l'acide cyclohexane carboxylique ;
- les acides dicarboxyliques cycloaliphatiques, tels que l'acide cyclohexane dicarboxylique ;
- les acides monocarboxyliques aromatiques, tels que l'acide benzoïque, les acides toluiques ou l'acide salicylique ;
- les acides dicarboxyliques aromatiques, tels que l'acide orthophtalique ou l'acide paraphtalique ;
- les acides tricarboxyliques aromatiques, tels que l'acide trimellitique ou l'acide trimésitique ;

et les acides tétracarboxyliques aromatiques, tels que l'acide pyromellitique, l'acide biphényle -4,4' tétracarboxylique, l'acide diphényléther -4,4' tétracarboxylique.

De manière préférée dans l'invention, l'acide carboxylique considéré est un acide monocarboxylique aliphatique de 2 à 22 atomes de carbone. Dans la formule (1) de l'ester, q est alors égal à zéro, p est égal à 1 et $R^2$ est un radical aliphatique monovalent de 1 à 21 atomes de carbone.

Les monoalcools (poly)sulfurés répondant à la formule (3) mentionnée précédemment peuvent être plus particulièrement des monoalcools primaires de formule $R^1$-$S_x$-$(CH_2)_n$-$CH_2OH$ (cas où $z = 1$ et $m = 0$), ou monoalcools secondaires de formule: $[R^1$-$S_x$-$(CH_2)n$-$]_2CHOH$ (cas où $z = 2$ et $m = 0$).

Dans les formules ci-dessus, le radical $R^1$ est de préférence un radical aliphatique monovalent, saturé ou insaturé de 1 à 12 atomes de carbone ; n est un nombre entier de 1 à 5; et x est un nombre supérieur ou égal à 1.

Parmi les monoalcools polysulfurés, c'est-à-dire ceux pour lesquels x est supérieur à 1, selon la méthode par laquelle ils ont été préparés, on considère d'une part des composés dits stoechiométriques, pour lesquels la valeur de x est une valeur entière, par exemple 2,3 ou plus : ce seront des monoalcools primaires disulfurés, trisulfurés, etc ; et des monoalcools secondaires bis(disulfurés), bis(trisulfurés), etc. On considère d'autre part des produits dits statistiques, qui consistent en des mélanges en proportions variées de composés pour lesquels x prend une valeur entière (1, 2 ou 3 etc), cette valeur différant d'un composé du mélange à un autre. La valeur de x indiquée pour le mélange est alors une valeur moyenne qui peut être entière ou non. Si elle est voisine de 2, de 3, etc, on parlera aussi dans ce cas de monoalcools primaires disulfurés, trisulfurés, etc, et de monoalcools secondaires bis(disulfurés), bis(trisulfurés), etc.

Comme exemples de monoalcools utilisables pour former les composés carboxyliques (poly)sulfurés de l'invention, on peut citer :

- des monoalcools primaires monosulfurés tels que t.Bu-S-$(CH_2)_2$-OH ; t.Bu-S-$(CH_2)_3$-OH ; t.Bu-S-$(CH_2)_6$-OH ; méthallyl -S-$(CH_2)_2$-OH ; méthallyl-S-$(CH_2)_3$-OH ; et méthallyl-S-$(CH_2)_6$-OH ;
- des monoalcools secondaires bis(monosulfurés) tels que (t.Bu-S-$CH_2$-$)_2CHOH$ ;
- des monoalcools primaires disulfurés (stoechiométriques ou statistiques) tels que t.Bu-$S_2$-$(CH_2)_2$-OH ; t.Bu-$S_2$-$(CH_2)_3$-OH ; t.Bu-$S_2$-$(CH_2)_6$-OH ; méthallyl-$S_2$-$(CH_2)_2$-OH ; méthallyl-$S_2$-$(CH_2)_3$-OH ; méthallyl-$S_2$-$(CH_2)_4$-OH ; méthallyl-$S_2$-$(CH_2)_5$-OH ; et méthallyl-$S_2$-$(CH_2)_6$-OH ;
- des monoalcools secondaires bis(disulfurés) tels que (t.Bu-$S_2$-$CH_2$-$)_2CHOH$ ;
- des monoalcools primaires trisulfurés, tels que t.Bu-$S_3$-$(CH_2)_2$-OH ;

etc.

Les monoalcools (poly)sulfurés considérés ci-dessus peuvent être préparés par diverses méthodes.

Ainsi, les monoalcools monosulfurés de formule $R^1$-S-$(CH_2)_n$- $CH_2OH$, où $R^1$ représente un radical aliphatique monovalent renfermant par exemple de 1 à 12 atomes de carbone, saturé ou insaturé, tel que le radical tertiobutyle ou le radical méthallyle, et n un nombre entier par exemple de 1 à 5, peuvent être obtenus par réaction d'un thiol $R^1SH$ avec un chloroalcool Cl-$(CH_2)_n$-$CH_2$-OH en milieu alcalin. De même, les alcools monosulfurés de formule $[R^1$-S-$(CH_2)_n]_2$CH-OH sont obtenus par réaction d'un thiol $R^1SH$ en milieu alcalin avec un chloro-alcool secondaire $[Cl(CH_2)_n]_2CHOH$.

Les monoalcools monosulfurés insaturés tels que méthallyl-S-$CH_2$-$CH_2OH$ peuvent en outre être obtenus par réaction du chlorure d'alkényle (par exemple de méthallyle) avec le mercapto-2 éthanol SH-$CH_2$-$CH_2$-OH en milieu alcalin.

Pour préparer les monoalcools disulfurés de formule $R^1$-$S_2$-$CH_2$-$CH_2$-OH, où $R^1$ représente un radical aliphatique monovalent, saturé ou insaturé, renfermant par exemple de 1 à 12 atomes de carbone, tel que le radical tertiobutyle ou le radical méthallyle, on fait réagir le chlorure de méthoxycarbonylsulfényle $CH_3$-O-

CO-S-Cl avec le mercapto-2 éthanol pour former le disulfure $CH_3$-O-CO-$S_2$-$CH_2$-$CH_2$-OH, que l'on fait réagir ensuite avec le thiol $R^1SH$.

Une méthode générale pour préparer les monoalcools polysulfurés de formule $R^1$-$S_x$-$(CH_2)_n$-$CH_2OH$ où $R^1$ est un radical aliphatique monovalent, saturé ou insaturé, renfermant par exemple de 1 à 12 atomes de carbone, n est un nombre entier de 1 à 5 et x un nombre supérieur à 1, consiste

(a) à préparer un thiolate $R^1$-$S_x$-Na par réaction du thiol $R^1SH$ avec de la soude en milieu aqueux ou alcoolique pour former le thiolate $R^1$-S-Na, puis par réaction de ce thiolate avec une proportion appropriée de soufre élémentaire, également en milieu aqueux ou alcoolique ;

puis (b) à faire réagir thiolate $R^1$-$S_x$-Na obtenu avec le chloroalcool Cl-$(CH_2)_n$-$CH_2OH$.

On obtient le monoalcool polysulfuré sous forme d'un mélange de composés pour lequel la valeur de x indiquée est une valeur moyenne (composé statistique).

Les monoalcools polysulfurés secondaires $(R^1$-$S_x$-$CH_2$-$)_2CHOH$ peuvent être obtenus par la même méthode dans laquelle on met en jeu le chloroalcool secondaire (Cl-$CH_2$-$)_2CHOH$.

En suivant cette méthode de préparation lorsgue $R^1$ représente un radical aliphatigue insaturé, par exemple méthallyle, à côté du monoalcool polysulfuré recherché, on obtient en général une proportion mineure de sulfures symétriques $R^1$-$S_x$-$R^1$, qui peuvent être éliminés par exemple par séparation sur gel de silice, ou maintenus dans le produit.

Pour préparer les monoalcools disulfurés de formule $R^1$-$S_x$-$(CH_2)_n$-$CH_2OH$ dans lesquels $R^1$ est un radical aliphatique insaturé, par exemple méthallyle, avec n par exemple de 2 à 5, on peut en outre procéder par réaction du thiolate $R^1$-S-Na avec l'hydroxyalkylthio sulfate de sodium $HO(CH_2)_n$-$CH_2$-$S_2O_3Na$. Par cette méthode, on obtient le composé disulfuré stoechiométrique. Ces monoalcools disulfurés sont nouveaux et constituent un des objets de l'invention.

Par ailleurs, on peut encore préparer les monoalcools trisulfurés stoechiométrigues de formule $R^1$-$S_3$-$CH_2$-$CH_2$-OH où $R_1$ représente un radical aliphatique monovalent, saturé ou insaturé, soit par réaction du thiol $R^1SH$ avec le chlorure de méthoxycarbonyldisulfanyle $CH_3$-O-CO-$S_2$-Cl suivie de la réaction du produit obtenu avec le mercapto-2 éthanol, en présence d'un catalyseur de type amine tel que la N-méthylmorpholine ; soit par réaction dans l'ordre inverse du mercapto-2 éthanol avec le chlorure de méthoxycarbonyldisulfanyle, suivie de la réaction du produit obtenu avec le thiol $R^1$-SH.

Les esters (poly)sulfurés de l'invention sont obtenus par condensation, dans les conditions usuelles d'estérification, des alcools (poly)sulfurés tels que décrits précédemment avec les acides carboxyliques appropriés.

Le catalysateur d'estérification peut être par exemple l'acide para-toluènesulfonique.

Lorsque l'on estérifie un acide polycarboxylique, on peut envisager de conduire l'estérification avec un mélange d'un ou plusieurs monoalcools (poly)sulfurés tels que décrits plus haut et d'un ou plusieurs monoalcools classiques non sulfurés, de manière à faire varier la teneur en soufre du mélange d'esters. Il convient alors qu'au moins une des fonctions carboxyliques de l'acide soit estérifiée par un monoalcool (poly)sulfuré. Comme monoalcools classiques on peut utiliser par exemple les monoalcools aliphatiques saturés de 1 à 12 atomes de carbone, c'est à dire du méthanol au dodécanol.

Les composés carboxyliques (poly) sulfurés de l'invention ont en général une teneur en soufre allant jusqu'à environ 60 % en poids. Ces composés présentent de bonnes proprietés antiusure et extrême-pression, et peuvent être avantageusement utilisés comme additifs pour les huiles lubrifiantes, minérales ou synthétiques, notamment pour les huiles d'engrenages et pour les huiles destinées au travail des métaux. La proportion de composé (poly)sulfuré dans l'huile pourra être par exemple de 0,05 à 20 % en poids, plus particulièrement de 0,2 à 5 % de poids.

Un des avantages des composés de l'invention dans leur utilisation comme additifs pour les lubrifiants est, comme indiqué plus haut, la possibilité de modifier leurs propriétés anti-usure et extrême-pression en faisant varier le nombre d'atomes de soufre dans les channes soufrées (valeur du nombre x). Ainsi, à concentration en soufre constante dans le lubrifiant, les composés à enchaînements polysulfures se montrent plus efficaces que les composés monosulfures; c'est à dire contenant un (ou plusieurs) atome(s) de soufre isolé(s).

C'est pourquoi les composés préférés dans l'invention sont ceux pour lesquels le nombre x a une valeur strictement supérieure à 1, plus particulièrement une valeur moyenne d'au moins 2.

Les exemples suivants illustrent l'invention.

**EXEMPLES** On décrit tout d'abord la préparation des alcools sulfurés qui seront utilisés pour préparer des esters (Exemples 1 à 7).

**Exemple 1** Préparation de l'alcool monosulfuré de formule:

t.Bu-S-$CH_2$-$CH_2$-OH

On dissout 10 g de soude en pastilles (0,25 mole-g) dans 100 $cm^3$ d'alcool éthylique ; après dissolution, on porte le mélange à 50 ° C, puis on ajoute 22,5 g de 2-méthyl-2 propanethiol (0,25 mole-g). A la fin de l'addition, on maintient la température à 50 ° C pendant encore 0,5 heure.

On ajoute ensuite goutte à goutte en 1 heure 14,33 g de chloroéthanol (0,2 mole-g) en maintenant la température à 50 ° C, puis on porte le mélange à la température de reflux du solvant durant 5 heures.

On refroidit le mélange, on ajoute 500 $cm^3$ d'eau et on extrait le produit visé par agitation du mélange précédent avec 100 $cm^3$ de dichlorométhane ; on décante, la phase organique récupérée est lavée à l'eau, séchée sur $Na_2SO_4$ anhydre, filtrée puis évaporée à 100 ° C sous pression réduite.

On recupère 23g d'un produit incolore visqueux, dont l'analyse élémentaire est la suivante :

| C % Masse | | H % Masse | | S % Masse | |
|---|---|---|---|---|---|
| théorie | touvé | théorie | trouvé | théorie | trouvé |
| 53,71 | 53,92 | 10,44 | 10,37 | 23,92 | 23,82 |

Les analyses RMN 13C et Infra-rouge confirment la structure du produit obtenu.

**Exemple 2** Préparation de l'alcool bis (monosulfuré) de formule :

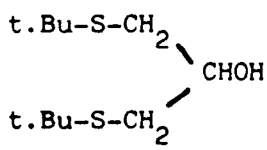

L'expérimentation de l'exemple 1 est reprise en substituant le chloroéthanol par une quantité molaire moitié moindre (0,1 mole-g : 12,9g) de 1,3-dichloropropanol, l'introduction de ce réactif étant effectuée en 1 heure à une température réactionnelle comprise entre 25 et 30 ° C.

Après réaction puis traitement, on récupère 22g d'un produit incolore visqueux, dont l'analyse élémentaire est la suivante :

| C % Masse | | H % Masse | | S % Masse | |
|---|---|---|---|---|---|
| théorie | trouvé | théorie | trouvé | théorie | trouvé |
| 55,90 | 55,95 | 10,16 | 10,25 | 27,16 | 27,32 |

Les analyses RMN 13C et Infra-rouge confirment la structure du produit obtenu.

**Exemple 3** Préparation de l'alcool monosulfuré de formule :

méthallyl-S-$(CH_2)_3$-OH

Dans une première étape, on prépare le 2-méthyl 1-propène 3-thiol en dissolvant 41,9g de thiourée (0,55 mole-g) dans 80 $cm^3$ de triéthylène glycol porté à 75 ° C, puis en ajoutant progressivement 45,25g (0,5 mole-g) de chlorure de méthallyle et en maintenant la température réactionnelle inférieure à 130 ° C.

On ajoute ensuite progressivement 94,5g de tétraéthylène pentamine (0,5 mole-g), le thiol formé qui distille (PE = 40 ° C) est recueilli et conservé.

Dans une seconde étape, on dissout 9,6g (0,24 mole-g) de NaOH pur dans 200 $cm^3$ d'alcool éthylique puis, après dissolution, on ajoute progressivement 21,1g (0,24 mole-g) de thiol préparé dans la première étape en maintenant la température réactionnelle au-dessous de 40 ° C, puis on porte le mélange à 50 ° C,

on maintient cette température pendant 0,5 heure puis on refroidit à 20 °C.

On ajoute alors progressivement 18,9g (0,2 mole-g) de 3-chloro 1-propanol puis le mélange est porté à la température du reflux, on maintient cette température pendant 6 heures, on refroidit et on filtre le mélange pour éliminer le NaCl formé.

La solution alcoolique filtrée est diluée avec 1000 $cm^3$ d'eau, l'alcool soufré est extrait avec du benzène, la solution organique récupérée est séchée sur $Na_2SO_4$ anhydre, filtrée et évaporée sous pression réduite. L'alcool soufré obtenu (25g) répond aux caractéristiques analytiques suivantes :

| C % Masse | | H % Masse | | S % Masse | |
|---|---|---|---|---|---|
| trouvé | théorie | trouvé | théorie | trouvé | théorie |
| 57,67 | 57,51 | 9,62 | 9,58 | 21,88 | 21,95 |

Les analyses Infra-rouge et RMN 13C confirment la structure chimique du produit visé :

$CH_2 = C(CH_3)-CH_2-S-(CH_2)_3-OH$

**Exemple 4** Préparation de l'alcool disulfuré de formule :

t.Bu-$S_2$-$CH_2$-$CH_2$-OH

Ce produit est préparé selon deux méthodes différentes.

Méthode A

Dans une première étape, on prépare du 2-hydroxy-éthyl-méthoxycarbonyldisulfure $CH_3$-O-C(O)-S-S-$CH_2$-$CH_2$-OH en utilisant la méthode de synthèse préconisée par FIELD et RAVICHANDRAN (J. Org. Chem, Vol.44 2624, 1979).

Dans une seconde étape, on mélange 35g de 2-hydroxy-éthyl-méthoxycarbonyldisulfure (0,208 mole-g) avec 50 $cm^3$ de méthanol et 1 $cm^3$ de triéthylamine. On ajoute ensuite 18,72g de 2-méthyl-2 propanethiol (0,208 mole-g) en 1 heure à 25 °C. Après évaporation du méthanol sous pression réduite, on récupère un produit liquide jaune.

Méthode B

L'expérimentation de l'exemple 1 est reprise, en ajoutant 8g de soufre élémentaire (0,25 at-g) à la solution alcoolique de 2-méthyl-2 propanethiolate de sodium et en chauffant à 50 °C pendant une heure supplémentaire, pour favoriser la formation du disulfure statistique.

Après addition du chloroéthanol puis traitements, on récupère un liquide jaune dont les caractéristiques analytiques sont voisines de celles du produit, non statistique, obtenu par la méthode A :

| Produit de la méthode | C % Masse | | H % Masse | | S % Masse | |
|---|---|---|---|---|---|---|
| | théorie | trouvé | théorie | trouvé | théorie | trouvé |
| A | 43,34 | 43,39 | 8,43 | 8,51 | 38,60 | 38,71 |
| B | | 44,08 | | 8,82 | | 38,25 |

L'analyse RMN 13C (qui montre en particulier la présence, dans le produit statistique obtenu par la méthode B, de sulfures en $S_1$, $S_2$, et $S_3$), et l'analyse Infra-rouge confirment la structure des produits obtenus.

**Exemple 5** Préparation de l'alcool disulfuré de formule :

t.Bu-$S_2$-$(CH_2)_3$ -OH

Dans une première étape, on dissout 54g (1,35 mole-g) de NaOH pur dans 2500 $cm^3$ d'éthanol, on

ajoute progressivement 117g (1,33 mole-g) de méthyl-2 propane thiol-2 sous vigoureuse agitation, on chauffe le mélange à 50 °C puis on ajoute 42,6g (1,33 atome-g) de soufre puis on refroidit le mélange à 20 °C.

Dans une seconde étape on ajoute progressivement au mélange précédent, 122,8g (1,3 mole-g) de chloro-3 propanol-1 puis le mélange est porté à la température du reflux, la suite de l'expérimentation est poursuivie dans les conditions de l'exemple 1.

L'alcool polysulfuré brut récupéré est purifié par chromatographie sur gel de silice ; une élution à l'hexane permet d'éliminer les polysulfures d'alkyle formés, et une élution à l'alcool méthylique permet de récupérer en solution le produit visé.

Après évaporation de l'alcool méthylique sous pression réduite on récupère un liquide jaune qui répond aux caractéristiques analytiques suivantes :

| C % Masse | | H % Masse | | S % Masse | |
|---|---|---|---|---|---|
| trouvé | théorie | trouvé | théorie | trouvé | théorie |
| 46,87 | 46,63 | 8,92 | 8,88 | 35,24 | 35,60 |

L'analyse Infra-rouge confirme la structure alcoolique attendue et l'analyse RMN 13C, montre qu'il s'agit d'un mélange d'alcools sulfurés dont le nombre d'atomes de soufre consécutifs est statistiquement égal à 2 comme indiqué dans la formule chimique suivante (x = 2) :

$(CH_3)_3$-C-$S_2$-$(CH_2)_3$-OH

**Exemple 6** Préparation de l'alcool disulfuré de formule :

Méthallyl-$S_2$-$(CH_2)_4$-OH

On prépare tout d'abord un hydroxyalkylthiosulfate en dissolvant 21,7g de 4-chloro 1-butanol (0,2 mole-g) dans 160 $cm^3$ de méthanol, on ajoute 40$cm^3$ d'eau, on porte le mélange à la température du reflux et on y ajoute 0,252 mole-g de thiosulfate de sodium dissous dans 100cm3 d'eau.

Le mélange est maintenu à ébullition pendant 2 heures, puis le méthanol est éliminé par évaporation. Après refroidissement, la phase aqueuse, préalablement lavée à l'hexane, est conservée.

On utilise ensuite 17,6g de 2-méthyl 1-propène 3-thiol (0,2 mole-g), que l'on transforme en thiolate de sodium selon la pro- cédure de l'exemple 3, mais en opérant en milieu aqueux.

On mélange enfin ces deux solutions à 0 °C, on y ajoute 100 $cm^3$ d'une solution aqueuse saturée de NaCl, on laisse revenir la température à 5 °C tout en agitant et l'on maintient cette température pendant une heure supplémentaire.

On procède alors à l'extraction de la partie organique au moyen d'éther éthylique : après lavage à l'eau, séchage sur $Na_2SO_4$ anhydre et évaporation de la phase organique sous pression réduite, on récupère 35g de produit liquide jaune pâle.

| C % Masse | | H % Masse | | S % Masse | |
|---|---|---|---|---|---|
| théorie | trouvé | théorie | trouvé | théorie | trouvé |
| 49,71 | 49,87 | 8,80 | 8,85 | 33,20 | 33,01 |

L'analyse RMN 13C et l'analyse Infra-rouge confirment la structure du produit obtenu.

**Exemple 7** Préparation de l'alcool trisulfuré de formule :

t.Bu-$S_3$-$CH_2$-$CH_2$-OH

Dans une première étape, on prépare du méthoxycarbonyl-tert-butyl trisulfure $CH_3$-O-C(O)-$S_3$-tert-butyle en utilisant la méthode de synthèse préconisée par BARANY et MOTT (Syntheses communi- cations 4, 657, (1984)).

Dans une seconde étape, on dissout 42,4g de $CH_3$-O-C(O)-$S_3$-tert-butyle (0,2 mole-g) dans 500 $cm^3$ de chloroforme, on ajoute 2g de N-méthylmorpholine et 15,6g de mercaptoéthanol : le mélange est agité à 20 °C pendant 24 heures.

Après traitement avec HCl 0,1 N, séparation des phases, séchage de la phase organique et évaporation du solvant sous pression réduite, on récupère 30g de produit liquide jaune visqueux.

| C % Masse | | H % Masse | | S % Masse | |
|---|---|---|---|---|---|
| théorie | trouvé | théorie | trouvé | théorie | trouvé |
| 36,33 | 36,81 | 7,06 | 7,45 | 48,54 | 48,12 |

Les analyses RMN 13C et Infra-rouge confirment la structure du produit obtenu.

**Exemples 8 à 15** Synthèses d'esters

On opère selon la méthode décrite ci-après. On introduit dans un réacteur muni d'un séparateur Dean-Stark, 21 mmoles d'alcool sulfuré, 26 mmoles d'acide (propanoïque ou laurique), 40 mg d'acide para-toluène sulfonique et 17 ml de cyclohexane. Le mélange est porté à reflux sous agitation et la manipulation est poursuivie jusqu'à l'obtention de la quantité requise d'eau. Après retour à la température ambiante, la phase organique est lavée avec une solution saturée de bicarbonate de sodium, à l'eau et avec une solution saturée de chlorure de sodium. Après évaporation de l'hexane on isole une huile de pureté suffisante (aucune trace d'alcool en RMN). Tous ces produits se caractérisent en analyse Infra-rouge par une bande très forte située vers 1740 $cm^{-1}$.

Les alcools sulfurés et les acides utilisés sont les suivants :

| **Exemple** | Acide carboxylique | Alcool sulfuré de l'exemple |
|---|---|---|
| **8** | Acide dodécanoïque | 1 |
| **9** | Acide propanoïque | 1 |
| **10** | Acide propanoïque | 2 |
| **11** | Acide dodécanoïque | 2 |
| **12** | Acide dodécanoïque | 3 |
| **13** | Acide dodécanoïque | 4 B |
| **14** | Acide propanoïque | 5 |
| **15** | Acide dodécanoïque | 6 A |

Les caractéristiques analytiques de ces produits sont réunies dans le tableau 1 ci-après.

Tableau 1

| Exemple | Caractéristiques des esters obtenus | | | | | |
|---|---|---|---|---|---|---|
| | C % Masse | | H % Masse | | S % Masse | |
| | théorie | trouvé | théorie | trouvé | théorie | trouvé |
| 8 | 68,34 | 68,84 | 11,39 | 11,88 | 10,14 | 9,78 |
| 9 | 56,82 | 57,34 | 9,47 | 9,69 | 16,87 | 16,11 |
| 10 | 57,51 | 58,21 | 9,58 | 10,25 | 21,95 | 21,12 |
| 11 | 66,01 | 66,67 | 11,00 | 11,57 | 15,37 | 14,87 |
| 12 | 69,50 | 69,97 | 10,97 | 11,25 | 9,77 | 9,65 |
| 13 | 62,05 | 62,67 | 10,34 | 10,65 | 18,42 | 18,11 |
| 14 | 50,82 | 51,33 | 8,47 | 8,87 | 27,16 | 26,67 |
| 15 | 64,15 | 64,76 | 10,16 | 10,41 | 17,14 | 17,00 |

**Exemple 16** Evaluation des propriétés extrême-pression et antiusure des additifs de l'invention.

On a réalisé des essais mettant en évidence les propriétés extrême-pression et antiusure des esters des exemples 8 et 12 à 15, à l'aide d'une machine 4 billes selon la procédure de l'ASTM D 2783, à des

concentrations telles que la teneur en soufre apportée par l'additif dans l'huile minérale SAE 80W90 soit égale à 0,22 % en masse : les résultats obtenus sont rassemblés dans le tableau 2.

On constate au vu des résultats que les additifs de l'invention possèdent de bonnes propriétés antiusure et extrême-pression, et que celles-ci peuvent être modifiées en fonction de la quantité de soufre élémentaire utilisée dans les synthèses.

On constate en particulier, qu'à concentration en soufre constante dans l'huile de base, les esters comportant des motifs polysulfures sont plus efficaces pour réduire l'usure que ceux comportant des motifs monosulfures, et que pour un motif mono ou polysulfure donné, les esters éthylèniques sont plus efficaces que les esters saturés, en particulier lorsque l'insaturation se trouve en position $\beta$ par rapport à la liaison C-S

$$(C=\underset{\beta}{C}-\underset{\alpha}{C}-S\sim\sim\sim),$$

d'où la possibilité de règler à volonté les performances mécaniques de ces produits.

Cette amélioration peut être mise à profit dans la formulation d'huiles lubrifiantes pour engrenages ou pour le travail des métaux.

TABLEAU 2

| Ester de l'exemple | Concentration en S dans l'additif % Masse | Concentration en additif dans l'huile % Masse | Concentration en S due à l'additif dans l'huile % Masse | MACHINE 4 BILLES | | | | | | |
|---|---|---|---|---|---|---|---|---|---|---|
| | | | | EXTREME-PRESSION | | | | USURE | | |
| | | | | Indice charge-usure | | Charge de soudure | | Diamètre d'empreinte (mm) | | |
| | | | | kgf | N | kgf | N | 40 kgf 392,4 N | 60 kgf 588,6 N | 80 kgf 784,8 N |
| huile seule | - | 0 | - | 22,50 | 220,7 | 160 | 1569,5 | 0,82 | 1,98 | 2,08 |
| 8 | 9,78 | 2,25 | 0,22 | 32,75 | 321,3 | 200 | 1962,0 | 0,39 | 0,56 | 1,10 |
| 13 | 18,11 | 1,21 | 0,22 | 45,1 | 442,4 | 200 | 1962,0 | 0,34 | 0,49 | 0,83 |
| 14 | 26,67 | 0,83 | 0,22 | 42,2 | 414,0 | 200 | 1962,0 | 0,33 | 0,53 | 0,91 |
| 12 | 9,65 | 2,28 | 0,22 | 40,5 | 397,3 | 200 | 1962,0 | 0,38 | 0,56 | 1,00 |
| 15 | 17,00 | 1,29 | 0,22 | 49,8 | 488,5 | 250 | 2452,5 | 0,32 | 0,51 | 0,78 |

## Revendications

1. Un composé carboxylique caractérisé en ce qu'il répond à la formule générale.

$$[R^3O\text{-}CO\text{-}]_qR^2\{\text{-}CO\text{-}O\text{-}(CH_2)_mCH_{3-z}[\text{-}(CH_2)_n\text{-}S_x\text{-}R^1]_z\}_p \quad (1)$$

dans laquelle $R^1$ représente un radical aliphatique monovalent, saturé ou insaturé, de 1 à 30 atomes de carbone ; x est un nombre égal ou supérieur à 1 ; z est égal à 1 ou 2 ; lorsque z est égal à 1, n est un nombre entier de 0 à 5 et m est un nombre entier de 0 à 5, avec m + n entier de 1 à 10 ; lorsque z est égal à 2, n est un nombre entier de 1 à 5 et m un nombre entier de 0 à 5, avec m + n entier de 1 à 10 ; $R^2$ est un radical hydrocarboné de 1 à 51 atomes de carbone et de valence p + q ; $R^3$ est un atome d'hydrogène ou un radical aliphatique monovalent de 1 à 30 atomes de carbone ; p est un nombre entier de 1 à 4 ; et q est un nombre entier de 0 à 3, avec p + q entier de 1 à 4.

2. Composé carboxylique selon la revendication 1, caractérisé en ce que, dans ladite formule, q est égal à zéro.

3. Composé carboxylique selon la revendication 2, caractérisé en ce que, dans ladite formule, p est égal à 1 et $R^2$ est un radical aliphatique monovalent de 1 à 21 atomes de carbone.

4. Composé carboxylique selon l'une des revendications 1 à 3, caractérisé et ce que, dans ladite formule, z est égal à 1, m est égal à zéro, $R^1$ représente un radical aliphatique monovalent de 1 à 12 atomes de carbone et x à une valeur d'environ 1 à 3.

5. Composé carboxylique selon l'une des revendications 1 à 3, caractérisée en ce que, dans ladite formule, z est égal à 2, m est égal à zéro, $R^1$ représente un radical aliphatique monovalent de 1 à 12 atomes de carbone et x a une valeur d'environ 1 à 3.

6. Composé carboxylique selon l'une des revendications 1 à 5, caractérisé en ce que le nombre x est strictement superieur à 1.

7. Composé carboxylique selon l'une des revendications 1 à 5, caractérisé en ce que le nombre x a une valeur moyenne d'au moins 2.

8. Procédé de préparation d'un compose carboxylique selon l'une des revendications 1 à 7, caractérisé en ce que l'on fait réagir dans des conditions d'estérification un acide mono ou polycarboxylique de formule

$$R^2\ [C(O)OH]_{p+q}$$

avec un alcool mono ou polysulfuré de formule

$$[R^1\text{-}S_x\text{-}(CH_2)_n\text{-}]_zCH_{3-z}\text{-}(CH_2)_m\text{-}OH$$

et éventuellement un alcool aliphatique hydrocarboné $R^3$ OH, où $R^1$, $R^2$, $R^3$, x, z, n, m, p et q ont les mêmes significations que dans la revendication 1 à 7.

9. A titre de produit intermédiaire le monoalcool disulfuré répondant à la formule

$$R^1\text{-}S_2\text{-}(CH_2)_n\text{-}CH_2OH$$

dans laquelle $R^1$ représente un radical aliphatique monovalent insaturé de 1 à 12 atomes de carbone et n est un nombre entier de 2 à 5.

10. Monoalcool disulfuré selon la revendication 9, caractérisé en ce que le radical $R^1$ est un radical méthallyle.

**11.** Une composition lubrifiante caractérisée en ce qu'elle comprend une proportion majeure d'huile lubrifiante minérale ou synthétique et une proportion mineure suffisante pour en améliorer les propriétés antiusure et extrême-pression, d'au moins un composé carboxylique selon l'une des revendications 1 à 7.

**12.** Composition lubrifiante selon la revendication 11, caractérisée en ce que ladite proportion mineure est de 0,05 à 20 % en poids.

## Claims

**1.** A carboxylic compound, characterised in that it is of the general formula

$$[R^3\text{-}OC(O)\text{-}]_q R^2\{\text{-}C(O)O\text{-}(CH_2)_m\text{-}CH_{3-z}\,[\text{-}(CH_2)_n\text{-}S_x\text{-}R^1]_z\,{}_p\} \quad (1)$$

wherein $R^1$ represents a saturated or unsaturated monovalent aliphatic radical with 1 to 30 carbon atoms; $\underline{x}$ is a number equal to or greater than 1; $\underline{z}$ equals 1 or 2; when $\underline{z}$ equals 1, $\underline{n}$ is an integer from 0 to 5 and $\underline{m}$ an integer from 0 to 5, with $\underline{m+n}$ an integer from 1 to 10; when $\underline{z}$ equals 2, $\underline{n}$ is an integer from 1 to 5 and $\underline{m}$ an integer from 0 to 5, with $\underline{m+n}$ an integer from 1 to 10; $R^2$ is a hydrocarbon radical with 1 to 51 carbon atoms and a valency of $\underline{p+q}$; $R^3$ is a hydrogen atom or a monovalent aliphatic radical with 1 to 30 carbon atoms; $\underline{p}$ is an integer from 1 to 4; and $\underline{q}$ is an integer from 0 to 3, with $\underline{p+q}$ being an integer from 1 to 4.

**2.** The compound of claim 1, characterised in that in said formula $\underline{q}$ equals zero.

**3.** The compound of claim 2, characterised in that in said formula $\underline{p}$ equals 1 and $R^2$ is a monovalent aliphatic radical with 1 to 21 carbon atoms.

**4.** The compound of any of claims 1 to 3, characterised in that in said formula $\underline{z}$ equals 1, $\underline{m}$ equals zero, $R^1$ represents a monovalent aliphatic radical with 1 to 12 carbon atoms and $\underline{x}$ has a value of approximately 1 to 3.

**5.** The compound of any of claims 1 to 3, characterised in that in said formula $\underline{z}$ equals 1, $\underline{m}$ equals zero, $R^1$ represents a monovalent aliphatic radical with 1 to 12 carbon atoms and $\underline{x}$ has a value of approximately 1 to 3.

**6.** The compound of any of claims 1 to 5, characterised in that the number $\underline{x}$ is strictly greater than 1.

**7.** The compound of any of claims 1 to 5, characterised in that the number $\underline{x}$ has a mean value of at least 2.

**8.** A method of preparing the carboxylic compound of any of claims 1 to 7, characterised in that a mono or polycarboxylic acid of the formula

$$R^2\,[C(O)OH]_{p+q}$$

is reacted under esterification conditions with a mono or polysulphurised alcohol of the formula:

$$[R^1\text{-}S_x\text{-}(CH_2)_n\text{-}]_z\,CH_{3-z}\text{-}(CH_2)_m\text{-}OH$$

and possibly with an aliphatic hydrocarbon alcohol $R^3OH$, where $R^1$, $R^2$, $R^3$, $\underline{x}$, $\underline{z}$, $\underline{n}$, $\underline{m}$, $\underline{p}$ and $\underline{q}$ have the same meanings as in claims 1 to 7.

**9.** As an intermediate product, the disulphurised monoalcohol of the formula

$$R^1\text{ - }S_2\text{-}(CH_2)_n\text{-}CH_2OH$$

where $R^1$ represents an unsaturated monovalent aliphatic radical with 1 to 12 carbon atoms and $\underline{n}$ is an integer from 2 to 5.

**10.** The disulphurised monoalcohol of claim 9, characterised in that the radical $R^1$ is a methallyl radical.

**11.** A lubricating composition, characterised in that it comprises a major proportion of mineral or synthetic lubricating oil and a minor proportion - sufficient to improve its anti-wear and extreme pressure properties - of at least one carboxylic compound according to any of claims 1 to 7.

**12.** The lubricating composition of claim 11, characterised in that said minor proportion is from 0.05 to 20% by weight.

## Patentansprüche

**1.** Carboxylverbindung, dadurch gekennzeichnet, daß sie der allgemeinen Formel

$$[R^3O\text{-}CO\text{-}]_qR^2\ \{\text{-}CO\text{-}O\text{-}(CH_2)_mCH_{3-z}[\text{-}(CH_2)_n\text{-}S_x\text{-}R^1]_z\}_p \qquad (1)$$

entspricht, worin $R^1$ einen gesättigten oder ungesättigten, einwertigen aliphatischen Rest mit 1 bis 30 Kohlenstoffatomen darstellt, x eine Zahl von größer oder gleich 1 ist, z gleich 1 oder 2 ist, wenn z gleich 1 ist, n eine ganze Zahl von 0 bis 5 und m eine ganze Zahl von 0 bis 5 ist, wobei m + n zusammen 1 bis 10 ist, wenn z gleich 2 ist, n eine ganze Zahl von 1 bis 5 und m eine ganze Zahl von 1 bis 5 ist, wobei m + n zusammen 1 bis 10 ist, $R^2$ ein Kohlenwasserstoffrest mit 1 bis 51 Kohlenstoffatomen und der Wertigkeit p + q ist, $R^2$ ein Wasserstoffatom oder ein einwertiger aliphatischer Rest mit 1 bis 30 Kohlenstoffatomen ist, p eine ganze Zahl von 1 bis 4 und q eine ganze Zahl von 0 bis 3 ist, wobei p + q zusammen 1 bis 4 ist.

**2.** Carboxylverbindung nach Anspruch 1, dadurch gekennzeichnet, daß in der Formel q gleich 0 ist.

**3.** Carboxylverbindung nach Anspruch 2, dadurch gekennzeichnet, daß in der Formel p gleich 1 ist und $R^2$ ein einwertiger aliphatischer Rest mit 1 bis 21 Kohlenstoffatomen ist.

**4.** Carboxylverbindung nach einem der Ansprüche 1 bis 3, dadurch gekennzeichnet, daß in der Formel z gleich 1 ist, m gleich 0 ist, $R^1$ einen einwertigen aliphatischen Rest mit 1 bis 12 Kohlenstoffatomen darstellt und x einen Wert von etwa 1 bis 3 besitzt.

**5.** Carboxylverbindung nach einem der Ansprüche 1 bis 3, dadurch gekennzeichnet, daß in der Formel z gleich 2 ist, m gleich 0 ist, $R^1$ einen einwertigen aliphatischen Rest mit 1 bis 12 Kohlenstoffatomen darstellt und x einen Wert von etwa 1 bis 3 besitzt.

**6.** Carboxylverbindung nach einem der Ansprüche 1 bis 5, dadurch gekennzeichnet, daß die Zahl x streng über 1 liegt.

**7.** Carboxylverbindung nach einem der Ansprüche 1 bis 5, dadurch gekennzeichnet, daß die Zahl x einen mittleren Wert von wenigstens 2 besitzt.

**8.** Verfahren zur Herstellung einer Carboxylverbindung gemäß einem der Ansprüche 1 bis 7, dadurch gekennzeichnet, daß man unter Veresterungsbedingungen eine Mono- oder Polycarbonsäure der Formel

$$R^2[C(O)OH]_{p+q}$$

mit einem mono- oder polysulfurierten Alkohol der Formel

$$[R^1\text{-}S_x\text{-}(CH_2)_n\text{-}]_zCH_{3-z}\text{-}(CH_2)_m\text{-}OH$$

und gegebenenfalls einem aliphatischen Kohlenwasserstoffalkohol $R^3OH$ umsetzt, worin $R^1$, $R^2$, $R^3$, x, z, n, m, p und q die gleiche Bedeutung wie in Anspruch 1 bis 7 besitzen.

**9.** Disulfurierter Monoalkohol, welcher der Formel

$R^1-S_2-(CH_2)_n-CH_2OH$

entspricht in der $R^1$ einen ungesättigten, einwertigen aliphatischen Rest mit 1 bis 12 Kohlenstoffatomen darstellt und n eine ganze Zahl von 2 bis 5 ist.

**10.** Disulfurierter Monoalkohol nach Anspruch 9, dadurch gekennzeichnet, daß der Rest $R^1$ ein Methallylrest ist.

**11.** Schmiermittelzusammensetzung, dadurch gekennzeichnet, daß sie eine größere Menge mineralisches oder synthetisches Schmieröl und eine kleinere Menge wenigstens einer Carboxylverbindung nach einem der Ansprüche 1 bis 7 umfaßt, die ausreicht, um die Antiverschleiß- und Hochdruckeigenschaften davon zu verbessern.

**12.** Schmiermittelzusammensetzung nach Anspruch 11, dadurch gekennzeichnet, daß die kleinere Menge 0,05 bis 20 Gew.% beträgt.